**Europäisches Patentamt**

**European Patent Office**

**Office européen des brevets**

(19)

(11) Veröffentlichungsnummer: **0 149 145 B1**

(12) **EUROPÄISCHE PATENTSCHRIFT**

(45) Veröffentlichungstag der Patentschrift:
09.03.88

(21) Anmeldenummer: **84115280.4**

(22) Anmeldetag: **12.12.84**

(51) Int. Cl.⁴: **C 07 C 7/08**

| E R R A T U M |
| --- |

(SEITE, SPALTE, ZEILE)
(PAGE, COLUMN, LINE)
(PAGE, COLONNE, LIGNE)

| DIE TEXTSTELLE :<br>TEXT PUBLISHED :<br>LE PASSAGE SUIVANT : | | | | LAUTET BERICHTIGT:<br>SHOULD READ :<br>DEVRAIT ETRE LU : |
| --- | --- | --- | --- | --- |
| "Element of Fractional Distillation" | 2 | 1 | 23/24 | "Elements of Fractional Distillation' |
| $C_4$-Fraktionen an Vinylacetylenen, | | 2 | 65 | $C_4$-Fraktionen an Vinylacetylen, |
| die leichter löslichen Kohlenwasser-stoffe ... | 3 | 3 | 56 | die schwerer löslichen Kohlenwasser-stoffe ... |

Tag der Entscheidung
über die Berichtigung )
Date of decision on ) **19.04.88**
rectification: ) .....................
Date de décision portant )
sur modification: )

Ausgabe- und Veröffentlichungstag: )
Issue and publication ) **15.06.88**
date: ) .....................
Date d'edition et de )
publication: )

Patbl.Nr.) **88/24**

EPB no:) .........

Bull. no:)

Europäisches Patentamt

European Patent Office

Office européen des brevets

(11) Veröffentlichungsnummer: **0 149 145**
**B1**

# (12) EUROPÄISCHE PATENTSCHRIFT

(45) Veröffentlichungstag der Patentschrift:
**09.03.88**

(21) Anmeldenummer: **84115280.4**

(22) Anmeldetag: **12.12.84**

(51) Int. Cl.⁴: **C 07 C 7/08**

(54) **Verfahren zur Trennung eines C4-Kohlenwasserstoffgemisches durch Extraktivdestillation.**

(30) Priorität: **23.12.83 DE 3346695**

(43) Veröffentlichungstag der Anmeldung:
**24.07.85 Patentblatt 85/30**

(45) Bekanntmachung des Hinweises auf die Patenterteilung:
**09.03.88 Patentblatt 88/10**

(84) Benannte Vertragsstaaten:
**AT BE DE FR GB IT NL SE**

(56) Entgegenhaltungen:
**US - A - 4 128 457**

(73) Patentinhaber: **BASF Aktiengesellschaft,
Carl-Bosch-Strasse 38, D-6700 Ludwigshafen (DE)**

(72) Erfinder: **Lindner, Alfred, Dr. Chem., Ringstrasse 30,
D-6712 Bobenheim-Roxheim (DE)**
Erfinder: **Volkamer, Klaus, Dr. Chem., Heidelberger
Ring 21, D-6710 Frankenthal (DE)**
Erfinder: **Hoffmann, Herwig, Dr. Chem.,
Knietschstrasse 21, D-6710 Frankenthal (DE)**
Erfinder: **Sandrock, Gerhard, Dr. Chem.,
Albrecht-Duerer-Ring 36 c, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Beschreibung

Die vorliegende Erfindung betrifft ein Verfahren zur Trennung eines $C_4$-Kohlenwasserstoffgemisches, welches im selektiven Lösungsmittel leichter und schwerer lösliche Kohlenwasserstoffe enthält, durch Extraktivdestillation mit Hilfe eines selektiven Lösungsmittels.

Die Extraktivdestillation ist ein bekanntes Verfahren zur Trennung von Gemischen, die durch übliche fraktionierte Destillation nicht leicht trennbar sind, z.B. wenn die zu trennenden Komponenten ein Azeotrop bilden oder geringe Unterschiede in den relativen Flüchtigkeiten besitzen. Bei der Extraktivdestillation wird in die Destillationskolonne eine solche Menge eines relativ schwer flüchtigen Lösungsmittels eingeführt, dass die Unterschiede in den relativen Flüchtigkeiten der zu trennenden Komponenten erhöht werden und damit eine destillative Trennung ermöglicht wird. Typische Anwendungsbeispiele für die Extraktivdestillation finden sich beispielsweise in C.S. Robinson et al «Element of Fractional Distillation», 4. Auflage McGraw-Hill Book Company, Inc., New York (1959), Seite 291.

Es ist bekannt, z.B. aus der DE-AS 15 68 902 oder DE-PS 11 63 795, ein $C_4$-Kohlenwasserstoffgemisch zur Gewinnung von 1,3-Butadien durch Extraktivdestillation unter Verwendung eines selektiven Lösungsmittels aufzutrennen.

Bei den bekannten Verfahren wird in der Extraktivdestillationszone eine relativ hohe Konzentration des selektiven Lösungsmittels, bezogen auf das sich in der Extraktivdestillationszone bildende Gemisch aus $C_4$-Kohlenwasserstoffen und selektivem Lösungsmittel, aufrechterhalten, um eine selektive Trennung der $C_4$-Kohlenwasserstoffe zu bewirken. Diese relativ hohe Konzentration des selektiven Lösungsmittels hat jedoch hohe Lösungsmittelumlaufmengen und damit entsprechend hohe Energieverbräuche hochwertiger Energie zur Folge.

Die vorliegende Erfindung soll nun eine Verbesserung der Arbeitsweise und Wirtschaftlichkeit der bekannten Verfahren bewirken.

Es wurde nun ein vorteilhaftes Verfahren gefunden zur Trennung eines $C_4$-Kohlenwasserstoffgemisches mit Hilfe eines selektiven Lösungsmittels bei dem man das $C_4$-Kohlenwasserstoffgemisch in einer Extraktivdestillationszone in ein die schwerer löslichen Kohlenwasserstoffe enthaltendes Kopfprodukt (Destillat) und einen die leichter löslichen Kohlenwasserstoffe und selektives Lösungsmittel enthaltenden Extrakt auftrennt, den als Sumpfprodukt abgezogenen Extrakt einer Lösungsmittelwiedergewinnungszone zuführt, in der der Extrakt in ein die Kohlenwasserstoffe enthaltendes Produkt und in das von den Kohlenwasserstoffen ganz oder teilweise befreite selektive Lösungsmittel aufgetrennt wird, welches dadurch gekennzeichnet ist, dass die Konzentration des selektiven Lösungsmittels in der Extraktivdestillationszone, bezogen auf das sich in der Extraktivdestillationszone bildende Gemisch aus $C_4$-Kohlenwasserstoffen und selektivem Lösungsmittel, durch Variation der Menge des Kohlenwasserstoffrückflusses am Kopf der Extraktivdestillationszone, des Drucks, der Lösungsmittel-Zulauftemperatur oder, falls das selektive Lösungsmittel Wasser enthält, auch durch Veränderung des Wassergehalts des selektiven Lösungsmittels in der Weise eingestellt wird, dass sie mindestens auf einem Boden bzw. bei Extraktivdestillationskolonnen mit Füllköperschüttungen mindestens an einem Punkt innerhalb der Füllkörperschüttung 75 Gew.-% unterschreitet.

Nach dem neuen Verfahren kann die Umlaufmenge des selektiven Lösungsmittels abgesenkt werden bei gleicher Trennleistung wie bei den bekannten mit höherer Lösungsmittelkonzentration arbeitenden Verfahren. Dies ist überraschend, da die für die Trennung wesentliche Selektivität bei Erniedrigung der Lösungsmittelkonzentration in der Extraktivdestillationszone absinkt. Durch die Erniedrigung der Lösungsmittelumlaufmenge können kleinere Apparate eingesetzt werden, so dass sich die Investitionskosten entsprechend verringern. Weiterhin können im Falle der Butan/Buten-Trennung die Wärmeverluste durch das Rückkühlen des rückgeführten selektiven Lösungsmittels auf die Eintrittstemperatur des Lösungsmittels in die Extraktivdestillation gering gehalten werden, indem die im rückgeführten Lösungsmittel nach Wärmetausch mit beladenem Lösungsmittel verbliebene Wärme zur Trennung in einer destillativ wirkenden Zone nutzbringend verwendet wird.

Das Verfahren der vorliegenden Anmeldung ist allgemein anwendbar zur Trennung von $C_4$-Kohlenwasserstoffgemischen, die verschieden ungesättigte Verbindungen enthalten. Bei derartigen Systemen ist die stärker gesättigte Verbindung die im selektiven Lösungsmittel schwerer lösliche Komponente und die weniger gesättigte Verbindung die im selektiven Lösungsmittel leichter lösliche Komponente. Bei Isomeren, wie z.B. einer acetylenischen Verbindung und einem Diolefin, ist die acetylenische Verbindung leichter löslich als das Diolefin.

Beispielsweise wird das Verfahren nach der Erfindung vorteilhaft zum Trennen eines 1,3-Butadien enthaltenden $C_4$-Kohlenwasserstoffgemisches angewendet.

Solche $C_4$-Kohlenwasserstoffgemische werden z.B. als $C_4$-Fraktionen bei der Herstellung von Ethylen und/oder Propylen durch thermisches Spalten einer Petroleumfraktion, z.B. von verflüssigtem Petroleumgas (LPG), Leichtbenzin (Naphtha), Gasöl als Kohlenwasserstoff-Fraktion erhalten. Weiterhin werden solche $C_4$-Fraktionen bei der katalytischen Dehydrierung von n-Butan und/oder n-Buten erhalten. Die $C_4$-Fraktionen enthalten in der Regel Butane, n-Buten, Isobuten, 1,3-Butadien, Vinylacetylen, Ethylacetylen, 1,2-Butadien und gegebenenfalls geringe Mengen an $C_5$-Kohlenwasserstoffen, wobei der 1,3-Butadiengehalt im allgemeinen 10 bis 80 Gew.-%, vorzugsweise 20 bis 70 Gew.-%, insbesondere 30 bis 60 Gew.-% beträgt, während der Gehalt der $C_4$-Fraktionen an Vinylacetylenen,

Ethylacetylen und 1,2-Butadien insgesamt 5 Gew.-% im allgemeinen nicht übersteigt. Bei der erfindungsgemässen Extraktivdestillation dieser $C_4$-Fraktionen werden im allgemeinen die gesättigten und einfach olefinisch ungesättigten $C_4$-Kohlenwasserstoffe wie Butane, n-Buten und Isobuten als Kopfprodukt der Extraktivdestillationszone und 1,3-Butadien neben weiteren im selektiven Lösungsmittel leichter löslichen Kohlenwasserstoffen wie Vinylacetylen, Ethylacetylen und 1,2-Butadien als Produkt der Lösungsmittelwiedergewinnungszone erhalten. Dieses als Produkt der Lösungsmittelwiedergewinnungszone erhaltene Butadien wird in der Regel zur Gewinnung eines Reinstbutadiens noch weiteren Reinigungsoperationen unterworfen.

Weitere geeignete 1,3-Butadien enthaltende $C_4$-Kohlenwasserstoffgemische, die nach dem erfindungsgemässen Verfahren vorteilhaft getrennt werden, sind Rohbutadiene, die im allgemeinen mindestens 90 Gew.-%, vorzugsweise mindestens 95 Gew.-%, insbesondere mindestens 98 Gew.-% 1,3-Butadien enthalten und daneben als Verunreinigungen im allgemeinen einen Gehalt an höheren Acetylenen, wie Vinylacetylen und Ethylacetylen und höheren Allenen wie 1,2-Butadien aufweisen. Solche Rohbutadiene werden z.B. durch Extraktivdestillation, beispielsweise nach dem erfindungsgemässen Verfahren, von aus der thermischen Spaltung von Petroleumfraktionen oder bei der katalytischen Dehydrierung von n-Butan und/oder n-Buten erhaltenen $C_4$-Fraktionen gewonnen. Bei der Trennung eines solchen Rohbutadiens nach dem erfindungsgemässen Verfahren wird 1,3-Butadien als im selektiven Lösungsmittel schwerer löslicher Kohlenwasserstoff als Kopfprodukt der Extraktivdestillationszone und die höheren Acetylene und zumindest ein Teil der höheren Allene als im selektiven Lösungsmittel leichter lösliche Kohlenwasserstoffe als Produkt der Lösungsmittelwiedergewinnungszone erhalten.

Weitere $C_4$-Kohlenwasserstoffgemische, die als Ausgangs-$C_4$-Kohlenwasserstoffgemische für das erfindungsgemässe Verfahren geeignet sind, sind z.B. Butane, n-Butene und Isobuten enthaltende Gemische, wie sie z.B. als Destillat (Raffinat) aus einer Butadienextraktionsanlage erhalten werden, Butane und n-Butene enthaltende Gemische, wie sie beispielsweise nach Trennung von Isobuten aus dem vorstehend beschriebenen Raffinat erhalten werden, Butane und 2-Buten enthaltende Gemische, wie sie z.B. aus Anlagen zur Dimerisierung von n-Butenen erhalten werden. Die Trennung der $C_4$-Kohlenwasserstoffe in das die leichter löslichen Kohlenwasserstoffe enthaltende Destillat und ein die leichter löslichen Kohlenwasserstoffe enthaltendes Produkt kann neben der bereits vorstehend beschriebenen Trennung zwischen 1,3-Butadien und den Butenen sowie zwischen 1,3-Butadien und den Acetylenen beispielsweise auch zwischen Butan und den 1-Butenen, zwischen Butan und 2-Butenen oder Butan und 1,3-Butadien erfolgen.

Geeignete selektive Lösungsmittel für das erfindungsgemässe Verfahren sind z.B. Butyrolacton, Nitrile wie Acetonitril, Propionitril, Methoxypropionitril, Ketone wie Aceton, Furfurol, N-alkylsubstituierte niedere aliphatische Säureamide, wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon. Im allgemeinen werden N-alkylsubstituierte niedere aliphatische Säureamide oder N-alkylsubstituierte cyclische Säureamide verwendet. Mit besonderem Vorteil werden von den Lösungsmitteln Dimethylformamid und insbesondere N-Methylpyrrolidon verwendet.

Es können jedoch auch als selektives Lösungsmittel Mischungen dieser Lösungsmittel untereinander, z.B. von N-Methylpyrrolidon mit Acetonitril, Mischungen dieser Lösungsmittel mit Colösungsmitteln wie Wasser und/oder tert.-Butylether, z.B. Methyl-tert.-butylether, Ethyl-tert.-butylether, Propyl-tert.-butylether, n- oder iso-Butyl-tert.-butylether, eingesetzt werden.

Es ist ein wesentliches Merkmal des erfindungsgemässen Verfahrens, dass die Konzentration des selektiven Lösungsmittels in der Extraktivdestillationszone, d.h. in der Zone zwischen Lösungsmittelzulaufstelle und Lösungsmittelwiedergewinnungszone, bezogen auf das sich in der Extraktivdestillationszone bildende Gemisch aus $C_4$-Kohlenwasserstoffen und selektivem Lösungsmittel, 75 Gew.-% mindestens auf einem Boden bei Böden enthaltenden Extraktivdestillationszonen bzw. bei Extraktivdestillationszonen mit Füllkörperschüttungen mindestens an einem Punkt innerhalb der Füllkörperschüttung unterschreitet. Im allgemeinen beträgt die Konzentration des selektiven Lösungsmittels 30 bis 75 Gew.-%, vorzugsweise 35 bis 65 Gew.-%, insbesondere 40 bis 60 Gew.%. Die Konzentration des selektiven Lösungsmittels in der Extraktivdestillationszone wird durch Variation der Menge des Kohlenwasserstoffrückflusses am Kopf der Extraktivdestillationszone, des Drucks, der Lösungsmittel-Zulauftemperatur oder, falls das Lösungsmittel Wasser enthält, auch durch Veränderung des Wassergehalts des Lösungsmittels eingestellt.

Das am Kopf der Extraktivdestillationszone erhaltene Destillat wird zweckmässig als Kopfabzug oder Seitenabzug abgezogen. Zur Entfernung von geringen Mengen von im Destillat enthaltenem selektivem Lösungsmittel wird das Destillat zweckmässig mit Wasser gewaschen bzw. in einer destillativ wirkenden Zone im Gegenstrom zu einem flüssigen Kohlenwasserstoffrücklauf geführt, wodurch das Lösungsmittel zurückgehalten wird.

Der absolute Druck in der Extraktivdestillationszone und der Wasserwaschzone bzw. der destillativ wirkenden Zone liegt im allgemeinen zwischen 1,5 bar und 9 bar, vorzugsweise zwischen 2 und 8 bar, insbesondere zwischen 3 und 7 bar.

Die Temperatur in der Extraktivdestillationszone ist abhängig von der Bodenzahl bzw. der Schütthöhe der Füllkörperschüttung und dem Druck. Im allgemeinen liegt sie an der Stelle der niedrigsten Lösungsmittelkonzentration zwischen 20 °C und 80 °C, vorzugsweise zwischen 40 °C und 70 °C.

In der Lösungsmittelwiedergewinnungszone kann der Druck gegenüber der Extraktivdestillationszone abgesenkt sein. Im allgemeinen beträgt bei dieser Arbeitsweise der Druck in der Lösungsmittelwiedergewinnungszone 1,2 bis 3 bar. Es kann jedoch auch vorteilhaft sein, insbesondere bei der Trennung eines Butane und Butene enthaltenden $C_4$-Kohlenwasserstoffgemischs, das Verfahren in der Weise durchzuführen, dass sich am Kopf der Extraktivdestillationszone eine destillativ wirkende Zone anschliesst und dass der Druck in der Lösungsmittelwiedergewinnungszone mindestens dem Druck der Extraktivdestillationszone und destillativ wirkenden Zone entspricht, wobei der Druck in der Extraktivdestillationszone und destillativ wirkenden Zone mindestens so hoch ist, dass die Kondensationstemperatur des Kopfproduktes der destillativ wirkenden Zone mindestens 30 °C beträgt. Vorzugsweise wird dabei die destillativ wirkende Zone zusätzlich zur Extraktivdestillationszone zur Trennung des Butane und Butene enthaltenden $C_4$-Kohlenwasserstoffgemisches verwendet. Dabei kann es weiter vorteilhaft sein, aus der Lösungsmittelwiedergewinnungszone nur teilweise von den Kohlenwasserstoffen befreites selektives Lösungsmittel abzuziehen, das anschliessend in die Extraktivdestillationszone zurückgeführt wird.

Die Extraktivdestillation kann in einer Kolonne durchgeführt werden. Bei grossen Bodenzahlen z.B. bei Kolonnen mit mehr als 100 praktischen Böden kann es vorteilhaft sein, die Extraktivdestillation in mehr als einer Kolonne, im allgemeinen in zwei Kolonnen auszuführen. Zweckmässig wird man bei der Verwendung von zwei Kolonnen die oberhalb des Punktes, an dem das $C_4$-Kohlenwasserstoffgemisch in die Extraktivdestillationszone eingeführt wird, liegende Absorptionsstufe in die erste Kolonne und die unterhalb des Einführungspunktes des Kohlenwasserstoffgemisches liegende Anreicherungsstufe in die zweite Kolonne verlegen, d.h. der Einführungspunkt für das Kohlenwasserstoffgemisch ist am Kopf der zweiten Kolonne oder vorzugsweise am Sumpf der ersten Kolonne. Vorzugsweise wird man zwischen Absorptionsstufe und Anreicherungsstufe keine Kompressionsstufe zwischenschalten, sondern solche Druckverhältnisse innerhalb der Extraktivdestillationszone aufrechterhalten, wie sie sich von selbst in der Extraktivdestillationszone bei Fehlen von Kompressions- und/oder Druckminderungsstufen innerhalb der extraktiven Destillationszone einstellen, so dass der Druck am Boden der Extraktivdestillationszone entsprechend dem üblichen Druckverlust in den Kolonnen mindestens dem Druck am Kopf der Extraktivdestillationszone entspricht. In der Regel beträgt die Druckdifferenz zwischen Kopf und Boden der Extraktivdestillationszone 0,1 bis 3, vorzugsweise 0,2 bis 2 bar.

Der aus der Extraktivdestillationszone als Sumpfprodukt abgezogene Extrakt wird einer Lösungsmittelwiedergewinnungszone zugeführt, in der der Extrakt in ein die Kohlenwasserstoffe enthaltendes Produkt und in das von den Kohlenwasserstoffen ganz oder teilweise befreite selektive Lösungsmittel aufgetrennt wird. Die Lösungsmittelwiedergewinnungszone kann z.B. als Ausgaser oder als Lösungsmittelabstreifer bzw. als Kombination von Ausgaser und Lösungsmittelabstreifer betrieben werden.

Der Lösungsmittelwiedergewinnungszone wird im allgemeinen Wärme zugeführt, beispielsweise über einen Aufkocher. Das als Sumpfprodukt der Lösungsmittelwiedergewinnungszone erhaltene, von den Kohlenwasserstoffen ganz oder teilweise befreite selektive Lösungsmittel wird zweckmässig in die Extraktivdestillationszone zurückgeführt.

Das nachstehende Beispiel dient der weiteren Erläuterung der Erfindung.

Beispiel

Eine Extraktivdestillation (vgl. Fig.) wurde mit N-Methylpyrrolidon als selektivem Lösungsmittel betrieben, das über Leitung 10 der mit Böden ausgestatteten Extraktivdestillationszone 2 in einer Menge von 3 kg/h zugeführt wurde. Das eingesetzte $C_4$-Kohlenwasserstoffgemisch, das über Leitung 1 der Extraktivdestillationszone 2 in einer Menge von 1 kg/h zugeführt wurde und das aus einer Buten-Dimerisierungsanlage stammte, hatte die folgende Zusammensetzung:

| | |
|---|---|
| $C_3$-Kohlenwasserstoffe | 0,76 Gew.-% |
| Butan | 47,68 Gew.-% |
| iso-Butan | 11,57 Gew.-% |
| iso-Buten | 0,83 Gew.-% |
| 1-Buten | 1,18 Gew.-% |
| 2-Buten-cis | 10,54 Gew.-% |
| 2-Buten-trans | 27,44 Gew.-%. |

In der destillativ wirkenden Zone 4 wurden Lösungsmittelspuren, die in dem am Kopf der Extraktivdestillationszone erhaltenen Destillat enthalten waren, zurückgehalten. Gleichzeitig diente die destillativ wirkende Zone zur weiteren destillativen Trennung des am Kopf der Extraktivdestillationszone erhaltenen Destillats, wodurch 2-Buten-cis in dem Destillat abgereichert und zurückgehalten wurde. Die Konzentration des Lösungsmittels auf den Böden der Extraktivdestillation wurde durch Variation der Kohlenwasserstoffrückflussmenge eingestellt. Zu diesem Zweck wurde ein Teil des über Leitung 5 abgezogenen und kondensierten Raffinats über Leitung 6 zurückgeführt. Über Leitung 7 wurde Butan-Raffinat abgezogen. Ein Butene enthaltender Strom wurde über Leitung 8 abgezogen. Das am Sumpf der Lösungsmittelwiedergewinnungszone 3 abgezogene selektive Lösungsmittel wurde über Leitungen 9 und 10 nach Wärmetausch zum Einstellen der Temperatur wieder zum Kopf der Extraktivdestillationszone zurückgeführt.

Bei 1 kg/h Kohlenwasserstoffrücklauf unterschritt die Lösungsmittelkonzentration 51 Gew.-% auf mehreren Böden. Der über Leitung 8 abgezogene Strom enthielt 75 Gew.-% Butene und 25 Gew.-% Butane und entsprach daher der Zusammensetzung eines Einsatzgemisches für die Anlage zur n-Buten-Dimerisierung. Das über Leitung 7 abgezogene Kopfprodukt wies einen Gehalt an Butanen von 75 Gew.-% auf. Erhöhte man den Kohlenwasserstoffrücklauf gar auf 2 kg/h unterschritt die Lösungsmittelkonzentration 47 Gew.-% auf mehreren Böden, wobei sich bei gleichem Gesamt-Butengehalt des Buten-Stromes 8 von 75 Gew.-% der Butangehalt im über Leitung 7 erhaltenen Kopfprodukt gar auf 82 Gew.-% erhöhte.

Überraschenderweise brauchte der Druck der Lösungsmittelwiedergewinnungszone 3 von 4,75 bar nicht gegenüber dem Druck der Extraktivdestillationszone 2 und destillativ wirkenden Zone 4 abgesenkt zu werden, um eine ausreichende Reinheit der Produkte zu erreichen. Eine Kompression der in der Lösungsmittelwiedergewinnungszone ausgestreiften Kohlenwasserstoffe war dadurch nicht erforderlich. Die Kondensation des Kopfproduktes konnte mit Kühlwasser durchgeführt werden. Kältemittel war nicht erforderlich.

Vergleichsbeispiel

Führte man den Versuch wie vorstehend beschrieben durch, wobei jedoch durch Einstellung eines Kohlenwasserstoffrücklaufs von 0,18 kg/h und Erhöhung der Temperatur des selektiven Lösungsmittels eine Lösungsmittelkonzentration von minimal 75,5 Gew.-% in der Extraktivdestillationszone eingestellt wurde, so wies das Kopfprodukt nur einen Butan-Gehalt von 65 Gew.-% auf, d.h. im Kopfprodukt gingen ca. 3/4 der im eingesetzten $C_4$-Kohlenwasserstoffgemisch enthaltenen Butene verloren, so dass nur ca. 1/4 der Butene im Butenprodukt erhalten wurde und diese Arbeitsweise daher wirtschaftlich nicht mehr interessant war.

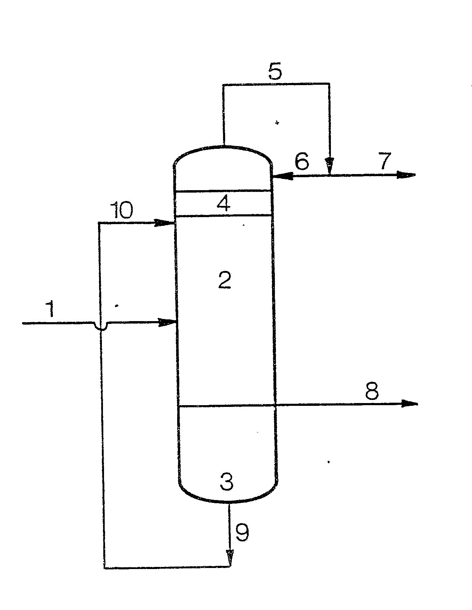

**Patentansprüche**

1. Verfahren zur Trennung eines $C_4$-Kohlenwasserstoffgemisches mit Hilfe eines selektiven Lösungsmittels, bei dem man das $C_4$-Kohlenwasserstoffgemisch in einer Extraktivdestillationszone in ein die schwerer löslichen Kohlenwasserstoffe enthaltendes Kopfprodukt (Destillat) und einen die leichter löslichen Kohlenwasserstoffe und selektives Lösungsmittel enthaltenden Extrakt auftrennt, den als Sumpfprodukt abgezogenen Extrakt einer Lösungsmittelwiedergewinnungszone zuführt, in der der Extrakt in ein die Kohlenwasserstoffe enthaltendes Produkt und in das von den Kohlenwasserstoffen ganz oder teilweise befreite selektive Lösungsmittel aufgetrennt wird, dadurch gekennzeichnet, dass die Konzentration des selektiven Lösungsmittels in der Extraktivdestillationszone, bezogen auf das sich in der Extraktivdestillationszone bildende Gemisch aus $C_4$-Kohlenwasserstoffen und selektivem Lösungsmittel, durch Variation der Menge des Kohlenwasserstoffrückflusses am Kopf der Extraktivdestillationszone, des Drucks, der Lösungsmittel-Zulauftemperatur oder, falls das selektive Lösungsmittel Wasser enthält, auch durch Veränderung des Wassergehalts des selektiven Lösungsmittels in der Weise eingestellt wird, dass sie mindestens auf einem Boden bzw. bei Extraktivdestillationskolonnen mit Füllkörperschüttungen mindestens an einem Punkt innerhalb der Füllkörperschüttung 75 Gew.-% unterschreitet.

2. Verfahren nach Anspruch 1, dadurch gekennzeichnet, dass es zur Trennung eines 1,3-Butadien enthaltenden $C_4$-Kohlenwasserstoffgemischs in ein gesättigte und einfach olefinisch ungesättigte $C_4$-Kohlenwasserstoffe enthaltendes Destillat und ein 1,3-Butadien und gegebenenfalls höhere Acetylene und 1,2-Butadien als im selektiven Lösungsmittel leichter lösliche Kohlenwasserstoffe enthaltendes Produkt angewendet wird.

3. Verfahren nach Anspruch 1, dadurch gekennzeichnet, dass es zur Trennung eines Rohbutadien in 1,3-Butadien als Destillat und in ein höhere Acetylene und gegebenenfalls 1,2-Butadien als im selektiven Lösungsmittel leichter lösliche Kohlenwasserstoffe enthaltendes Produkt angewendet wird.

4. Verfahren nach Anspruch 1, dadurch gekennzeichnet, dass es zur Trennung eines Butane und Butene enthaltenden $C_4$-Kohlenwasserstoffgemischs in ein Butane enthaltendes Destillat und ein die Butene als im selektiven Lösungsmittel leichter lösliche Kohlenwasserstoffe enthaltendes Produkt angewendet wird.

5. Verfahren nach Anspruch 4, dadurch gekennzeichnet, dass sich am Kopf der Extraktivdestillationszone eine destillativ wirkende Zone anschliesst und dass der Druck in der Lösungsmittelwiedergewinnungszone mindestens dem Druck der Extraktivdestillationszone und destillativ wirkenden Zone entspricht, wobei der Druck in der Extraktivdestillationszone und destillativ wirkenden Zone mindestens so hoch ist, dass die Kondensationstemperatur des Kopfproduktes der destillativ wirkenden Zone mindestens 30 °C beträgt.

6. Verfahren nach Anspruch 5, dadurch gekennzeichnet, dass die destillativ wirkende Zone zusätzlich zur Extraktivdestillationszone zur Trennung des Butane und Butene enthaltenden $C_4$-Kohlenwasserstoffgemischs verwendet wird.

7. Verfahren nach Anspruch 6, dadurch gekennzeichnet, dass das aus der Lösungsmittelwiedergewinnungszone abgezogene und rückgeführte selektive Lösungsmittel nur teilweise von den Kohlenwasserstoffen befreit wird.

**Revendications**

1. Procédé de séparation d'un mélange d'hydrocarbures en $C_4$ à l'aide d'un solvant sélectif, dans lequel on sépare le mélange d'hydrocarbures en $C_4$, dans une zone de distillation extractive, en un produit de tête (distillat) contenant les hydrocarbures peu solubles et en un extrait contenant les hydrocarbures plus solubles et du solvant sélectif, on amène l'extrait soutiré comme produit de fond à une zone de récupération du solvant, dans laquelle l'extrait est séparé en un produit contenant les hydrocarbures et en le solvant sélectif débarrassé en tout ou partie des hydrocarbures, procédé caractérisé par le fait que par modification de la quantité de reflux d'hydrocarbures à la tête de la zone de distillation extractive, de la pression, de la température d'amenée du solvant ou, dans le cas où le solvant sélectif contient de l'eau, également par modification de la teneur en eau du solvant sélectif, la concentration du solvant sélectif dans la zone de distillation extractive, rapportée au mélange d'hydrocarbures en $C_4$ et de solvant sélectif se formant dans la zone de distillation extractive est réglée de manière à ce qu'elle soit inférieure à 75% en poids sur au moins un plateau ou, pour des colonnes de distillation extractive à corps de garnissage, en au moins un point au sein du garnissage.

2. Procédé suivant la revendication 1, caractérisé par le fait qu'il est utilisé pour séparer un mélange d'hydrocarbures en $C_4$ contenant du 1,3-butadiène en un distillat contenant des hydrocarbures en $C_4$ saturés et des hydrocarbures en $C_4$ à une seule insaturation oléfinique, et en un produit contenant du 1,3-butadiène et éventuellement des acétylènes supérieurs et du 1,2-butadiène en tant qu'hydrocarbures plus solubles dans le solvant sélectif.

3. Procédé suivant la revendication 1, caractérisé par le fait qu'il est utilisé pour séparer un butadiène brut en 1,3-butadiène en tant que distillat et en un produit contenant des acétylènes supérieurs et éventuellement du 1,2-butadiène en tant qu'hydrocarbures plus solubles dans le solvant sélectif.

4. Procédé suivant la revendication 1, caractérisé par le fait qu'il est utilisé pour séparer un mélange d'hydrocarbures en $C_4$ contenant des butanes et des butènes en un distillat contenant des butanes et en un produit contenant les butènes en tant qu'hydrocarbures plus solubles dans le solvant sélectif.

5. Procédé suivant la revendication 4, caractérisé par le fait qu'à la suite de la tête de la zone de distillation extractive est prévue une zone à effet distillatoire et que la pression dans la zone de récupération du solvant correspond au moins à la pression de la zone de distillation extractive et de la zone à effet distillatoire, la pression dans la zone de distillation extractive et la zone à effet distillatoire étant, au moins, assez élevée pour que la température de condensation du produit de tête de la zone à effet distillatoire s'élève à 30 °C au moins.

6. Procédé suivant la revendication 5, caractérisé par le fait que la zone à effet distillatoire est utilisée additionnellement à la zone de distillation extractive pour la séparation du mélange d'hydrocarbures en $C_4$ contenant des butanes et des butènes.

7. Procédé suivant la revendication 6, caractérisé par le fait que le solvant sélectif, soutiré de la zone de récupération du solvant et recyclé, n'est que partiellement débarrassé des hydrocarbures.

**Claims**

1. A process for the separation of a $C_4$-hydrocarbon mixture with the aid of a selective solvent, in which the $C_4$-hydrocarbon mixture is separated, in an extractive distillation zone, into a top product (distillate), which contains the more sparingly soluble hydrocarbons, and an extract which contains the more readily soluble hydrocarbons and the selective solvent, the extract taken off as the bottom product is fed to a solvent recovery zone, in which the extract is separated into a product, which contains the hydrocarbons, and the selective solvent which has been partially or completely freed from the hydrocarbons, wherein the concentration, based on the $C_4$-hydrocarbon/selective solvent mixture formed in the extractive distillation zone, of the selective solvent in the said zone is adjusted by varying the amount of hydrocarbon reflux at the top of the extractive distillation zone, the pressure or the solvent feed temperature or, if the selective solvent contains water, also by varying the water content of the selective solvent, in a manner such that it falls below 75% by weight on one or more trays or, in the case of a packed extractive distillation column, at one or more points within the packing.

2. A process as claimed in claim 1, which is used for separating a buta-1,3-diene-containing $C_4$-hydrocarbon mixture into a distillate, containing saturated and mono-olefinically unsaturated $C_4$-hydrocarbons, and a product containing buta-1,3-diene, with or without higher acetylenes and buta-1,2-diene, as hydrocarbons which are more readily soluble in the selective solvent.

3. A process as claimed in claim 1, which is used for separating a crude butadiene into a buta-1,3-diene as a distillate, and a product containing higher acetylenes, with or without buta-1,2-diene, as hydrocarbons which are more readily soluble in the selective solvent.

4. A process as claimed in claim 1, which is used for separating a $C_4$-hydrocarbon mixture containing butanes and butenes into a distillate containing butanes, and a product containing the butenes as hydrocarbons which are more readily soluble in the selective solvent.

5. A process as claimed in claim 4, wherein the top of the extractive distillation zone is connected to a distillation zone, and the pressure in the solvent recovery zone is not less than that of the extractive distillation zone and the distillation zone, the pressure in the two last mentioned zones being no lower than that required for the condensation temperature of the top product of the distillation zone to be 30 °C or higher.

6. A process as claimed in claim 5, wherein the distillation zone is used in addition to the extractive distillation zone for the separation of the $C_4$-hydrocarbon mixtures contaning butanes and butenes.

7. A process as claimed in claim 6, wherein the selective solvent taken off from the solvent recovery zone and recycled is only partialoy freed from the hydrocarbons.